# EUROPEAN PATENT APPLICATION

(11) **EP 3 789 083 A1**
(43) Date of publication of application: **10.03.2021**
(21) Application number: 19199821.0
(22) Date of filing: 26.09.2019
(51) Int. Cl.: A61N 1/375

(54) **ADHESIVE-BASED ANCHORING FOR IMPLANTABLE MEDICAL DEVICES**

(30) Priority: 06.09.2019 US 201962896591 P
(71) Applicant: BIOTRONIK SE & Co. KG, 12359 Berlin (DE)
(72) Inventor: Taff, Brian M., Portland, OR 97217 (US); Austin, Eric, Portland, OR 97221 (US); Becker, Andreas, Wilsonville, OR 97070 (US)
(74) Representative: Galander, Marcus

(57) **Abstract**

The present disclosure relates to a medical device delivery system (1), comprising an implantable medical device (2) and a delivery device (3) for delivering the implantable medical device (2) to an implantation site within a body of a patient, wherein the medical device delivery system (1) comprises a reservoir (4) for accommodating an adhesive (5) in a liquid state, wherein the medical device delivery system (1) is configured to discharge the liquid adhesive (5) from the reservoir (4) so as to bond the implantable medical device (2) to tissue (6) of the patient at the implantation site.

## Description

The present disclosure relates to a medical implant delivery system as well as to a method for anchoring an implantable medical device to tissue of a patient.

Various sites within the human anatomy present significant challenges for the known anchoring strategies employed by implantable medical devices. Legacy anchor designs have typically employed screw-based, tine-based, or compression-based concepts which present notable risks for physiologic perforation, detrimental blood flow modifications, promotion of necrotic tissue responses, and device dislodgement in cases where implantable medical devices are intended for attachment to thin tissues.

Particularly, anchoring implantable medical devices into thin tissue presents a variety of risks for patient wellbeing that include perforation (the side-effects which may include cardiac tamponade), restricted blood flow to healthy tissue (often leading to the promotion of necrotic tissue responses), and device dislodgement (potentially freeing the implant to roam within the vasculature subject to impinging blood flow). In the specific context of intracardiac (e.g. leadless) pacemaker applications, the need for such anchoring becomes especially acute in considering development needs for implants stationed within the patient's right atrium. Such implants will ultimately play a critical role in supporting, at least, both AAI(R) and DDD(R) therapies where pacing within the RA is necessary for the delivery of appropriate bradycardia management.

Particularly, document US 2018/0326215 A1 disclosed fixation mechanisms for attaching an implant to cardiac tissue such as active fixation tines, screws, clamps, or adhesive members.

Furthermore, document GB 2 412 069 A discloses a stimulator that is adapted for attachment by the use of a physiologically compatible adhesive.

Based on the above it is an objective to provide an improved means for stable and robust mechanical engagement between tissue, particularly thin tissue, and an implantable medical device that avoids risks for device/anatomy separation. Particularly, it is an objective to provide such a means that also enables a stable engagement that preserves viable blood perfusion to the attached and surrounding tissue, avoiding the promotion and growth of necrotic tissue. Furthermore, particularly, it is an objective to provide a means for engagement that mitigates, and/or avoids altogether, the risk of an implantable medical device anchor piercing completely through the thin tissue as part of standard device placement procedures.

In one aspect, a medical device delivery system is disclosed, comprising:
- an implantable medical device, and
- a delivery device (e.g. a catheter) for delivering the implantable medical device to an implantation site within a body of a patient,
wherein the medical device delivery system comprises a reservoir for accommodating an adhesive in a liquid state, wherein the medical device delivery system is configured to discharge a liquid adhesive from the reservoir so as to bond the implantable medical device to tissue of the patient at the implantation site.

Also, an implantable medical device is provided. The comprising implantable medical device comprises a reservoir for accommodating an adhesive in a liquid state. The implantable medical device is configured to discharge the liquid adhesive from the reservoir so as to bond the implantable medical device to tissue of the patient at the implantation site.

Particularly, a suitable liquid that can be used in the framework of the present disclosure is a rapidly curing medical grade adhesive. Furthermore, particularly, the curing of a liquid adhesive used in the present disclosure may be instated through the application of incident UV energy.

According to an embodiment, the reservoir is arranged in the implantable medical device. Alternatively, the reservoir may be arranged in the delivery device.

According to an embodiment, the implantable medical device comprises a distal end portion, wherein the reservoir is arranged in the distal end portion of the implantable medical device.

Furthermore, according to an embodiment, for discharging said liquid adhesive, the reservoir is fluidly connected to at least one openable outlet formed in a distal end portion of a casing of the implantable medical device.

Particularly, the outlet can be formed by a predetermined breaking point or line of the casing such as a perforation of the casing.

Further, according to an embodiment, the at least one outlet is configured to be opened by applying a pressure on said distal end portion of the casing. Such pressure can lead to a deformation of the reservoir which increases the pressure of the liquid adhesive in the reservoir and may cause the outlet to tear open in a defined manner.

For applying said pressure, the distal end portion of the casing may comprise at least one protrusion protruding from a surface of the distal end portion of the casing.

According to an alternative embodiment, the at least one outlet is closed by means of an elongated flexible closure that is configured to be pulled from a first position in which the at least one outlet is closed by the closure into a second position in which the outlet is open, i.e., set free by the closure. Particularly, such a pulling operation can be carried out via a suitable elongated member guided by the delivery device.

According to another alternative embodiment, the implantable medical device comprises a rotatable closure that closes the at least one outlet, wherein the rotatable closure is configured to be rotated with respect to the casing of the implantable medical device to open the at least one outlet. Particularly, in an embodiment, the rotatable closure comprises protrusions protruding from a surface of the rotatable closure, which protrusions are configured to engage with tissue of the patient at the implantation site to provide abutments so that the rotatable closure can be rotated with respect to the casing of the implantable medical device by rotating the casing when the protrusions engage said tissue. Particularly, rotating the casing of the implantable medical device may be achieved by rotating the implantable medical device using the delivery device.

According to yet another alternative embodiment, the implantable medical device comprises an elongated pivotable closure that closes the at least one outlet, wherein the pivotable closure is configured to be pivoted with respect to the casing of the implantable medical device to open the at least one outlet. Particularly, the pivotable closure is configured to engage with tissue of the patient at the implantation site so that the pivotable closure can be pivoted with respect to the casing of the implantable medical device by rotating the casing when the pivotable closure engages said tissue. Also here rotating the casing of the implantable medical device may be achieved by rotating the implantable medical device using the delivery device.

According to a further alternative embodiment, the delivery device comprises at least one displaceable puncturing member that is configured to be displaced along the delivery device so as to puncture the distal end portion of the casing to form at least one outlet of reservoir through which said liquid adhesive is dischargeable.

According to a further alternative embodiment, the delivery device comprises at least one channel forming at least a portion of said reservoir, wherein the at least one channel comprises at least one opening at a distal end of the delivery device to discharge liquid adhesive on the distal end portion of the implantable medical device and/or on tissue of the patient at the implantation site to bond the medical implant device to said tissue.

Particularly, according to an embodiment, the delivery device comprises an optical fiber having an end portion arranged at the distal end of the delivery device to irradiate said applied adhesive with UV light coupled into the optical fiber so as to cure said applied liquid adhesive.

Furthermore, according to an embodiment, the implantable medical device is an intracardiac pacemaker, wherein the pacemaker comprises a pacing electrode that is arranged on the distal end portion of the casing of the implantable medical device. Such an intracardiac pacemaker is also denoted as implantable leadless pacemaker. Particularly, according to an embodiment, the intracardiac pacemaker comprises a collar that extends around the pacing electrode. Particularly, the collar can comprise a steroid. The steroid may be affixed to the distal end of the intracardiac pacemaker by adhesively attaching a silicone rubber component to the intracardiac pacemaker that has the steroid embedded within. This affixed silicon rubber component holds the steroid in place on the device and allows for drug elution over time. Alternatively, the implantable medical device may be an implantable (e.g. vascular) sensor.

Further, according to an embodiment, the casing comprises a circumferential protrusion that protrudes from a surface of the distal end portion of the casing and extends around the pacing electrode, wherein the circumferential protrusion is arranged between the pacing electrode and the at least one outlet to hinder liquid adhesive from contacting the pacing electrode upon discharging of the liquid adhesive through the at least one outlet.

According to a further aspect, a method for anchoring an implantable medical device to tissue of a patient at an implantation site using a medical implant delivery system according to the present disclosure is disclosed, wherein the method comprises the steps of: positioning the medical device at the implantation site via the delivery device, and discharging liquid adhesive out of the reservoir to bond the medical device to the tissue.

According to an embodiment of the method, the method comprises the further step of: irradiating UV light onto the liquid adhesive to cure the adhesive.

In the following, embodiment and further features shall be described with reference to the Figures, wherein
- Fig. 1: shows an embodiment of a medical device delivery system for enabling adhesive-based anchoring of an implantable medical device, wherein particularly subject to deforming a distal end portion of a casing of the device, a series of pre-perforated/pre-thinned regions in the distal end portion of the casing rupture to release a liquid medical-grade adhesive capable of fixing the implantable medical device in place;
- Fig. 2: shows a further embodiment of a medical device delivery system, wherein the adhesive reservoir comprises at least one outlet that is closed by a pullable elongated closure;
- Fig. 3: shows a further embodiment of a medical device delivery system, wherein the medical device comprises a rotatable closure for closing the at least one outlet of the adhesive reservoir;
- Fig. 4: shows a further embodiment of a medical device delivery system, wherein the medical device comprises at least one elongated pivotable closure for closing the at least one outlet of the adhesive reservoir;
- Fig. 5: shows a further embodiment of a medical device delivery system, wherein the catheter comprises at least one puncturing member for puncturing the adhesive reservoir of the implantable medical device;
- Fig. 6: shows a further embodiment of a medical device delivery system, wherein the catheter comprises at least one channel to dispense adhesive for device anchoring; and
- Fig. 7: shows a further embodiment of a medical device delivery system, wherein the catheter comprises integrated UV fiber optic for excitation and curing of the device anchoring adhesive.

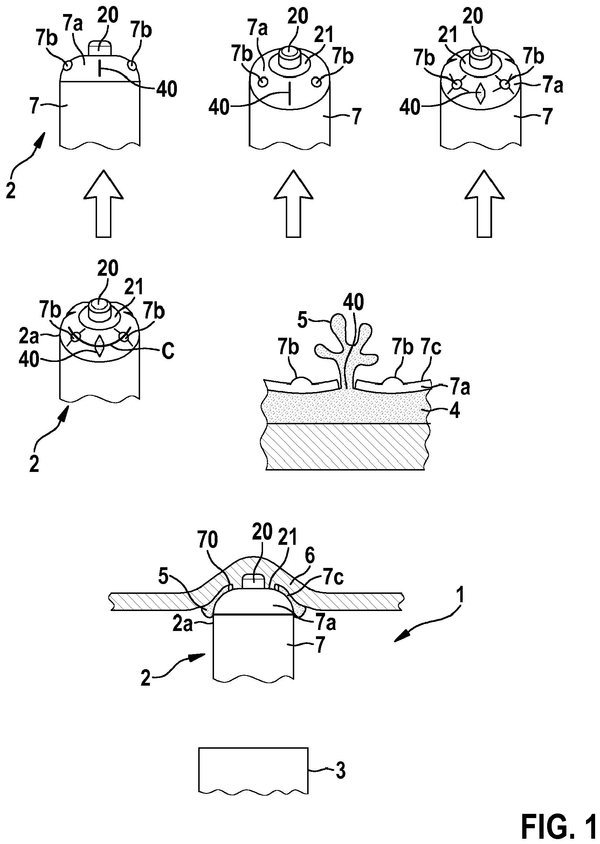

Fig. 1 shows an embodiment of a medical device delivery system 1 comprising an implantable medical device 2 (e.g. an intracardiac pacemaker 2), and a delivery device 3 (e.g. a catheter 3) for delivering the implantable medical device 2 to an implantation site within a body of a patient. Furthermore, the medical device delivery system 1 comprises a reservoir 4 for accommodating an adhesive 5 in a liquid state, wherein the medical device delivery system 1 is configured to discharge the liquid adhesive 5 from the reservoir 4 so as to bond the implantable medical device to tissue 6 of the patient at the implantation site. According to the embodiment shown in Fig. 1, the reservoir 4 is arranged in the implantable medical device. Particularly, the latter comprises a casing 7 housing the components of the device 2 and having a compliant distal end portion 7a to house the reservoir 4 for the liquid adhesive 5. Particularly, the adhesive 5 can be stationed behind a wall portion of the distal end portion 7a of the casing 7 in the form of a compliant membrane that incorporates at least one pre-perforated/pre-thinned material region 40 to form at least one openable outlet 40 that, subject to an appropriate application of stress, opens to deploy the liquid adhesive 5. As shown in Fig. 1, the casing 7 can comprise multiple outlets 40 of this kind. To assist in the adhesive deployment process, the compliant distal end portion 7a of the casing 7 can also comprise at least one or a series of protrusions 7b that protrude from a surface 7c of the distal end portion 7a of the casing 7 of the implantable medical device 2 and are positioned to engage with patient anatomy. Hence, when the medical device 2 is pressed against the targeted tissue anchoring site, these protrusions 7b deform the distal and portion 7a of the casing 7 to instate pressure on the underlying contained adhesive 5 residing in the reservoir 4. The distal end portion 7a of the casing 7 then responds to relieve this pressure by deforming and causing the at least one or the multiple pre-perforated/pre-thinned outlets 40 to tear open and release the liquid adhesive 5. This sequence of events is highlighted in stepwise fashion in the top portion of Fig. 1 as one marches from left to right.

The middle portion of Fig. 1 explicitly details this outlet opening and adhesive deployment response. The cross-section demarked with the solid line is detailed in side view to show the expulsion of the liquid adhesive 5 out of the reservoir 4 in the distal end portion 2a of the medical device 2 subject to depressing the protrusions 7b. For illustrative simplicity, the drawn cross-section does not include any internal baffles or features which might aid in amplifying the pressures exerted upon the pre-perforated/pre-thinned outlet features 40, but the present disclosure does not exclude the possibility of incorporating such design elements.

According to a preferred embodiment, the implantable medical device 2 is an intracardiac pacemaker 2 and comprises at the distal end portion 7a of its casing 7 a pacing electrode 20 (may also be used for sensing) as well as a steroid reservoir in the form of a collar 21 (for managing inflammatory responses and ostensibly lowering the pacing capture thresholds) extending around the pacing electrode 20. Preferably, the anchoring of the medical device 2 by establishing a bond to tissue 6 of the patient by means of the adhesive 5 should not disrupt the functionality of such elements like the pacing electrode 20 and the steroid collar 21. The bottom portion of Fig. 1 shows a cross-sectional depiction of the distal end portion 2a of the pacemaker 2 when anchored against thin-walled tissue 6 using the deployed adhesive 5. Here, an optional dam feature in form of a circumferential protrusion 70 surrounding the collar 21 and the pacing electrode 20 is provided on the distal end portion 7a of the casing 7 to prevent adhesive 5 from covering the steroid or electrode surfaces.

The present disclosure may be further extended through the use of not only rapid self-curing medical grade adhesives 5, but through the use of UV adhesives 5 which may be cured using either a separate fiber optic enabled catheter (independent of the device implantation catheter), or by means of enhancements to the implantation catheter 3 used to place and anchor the implant 2.

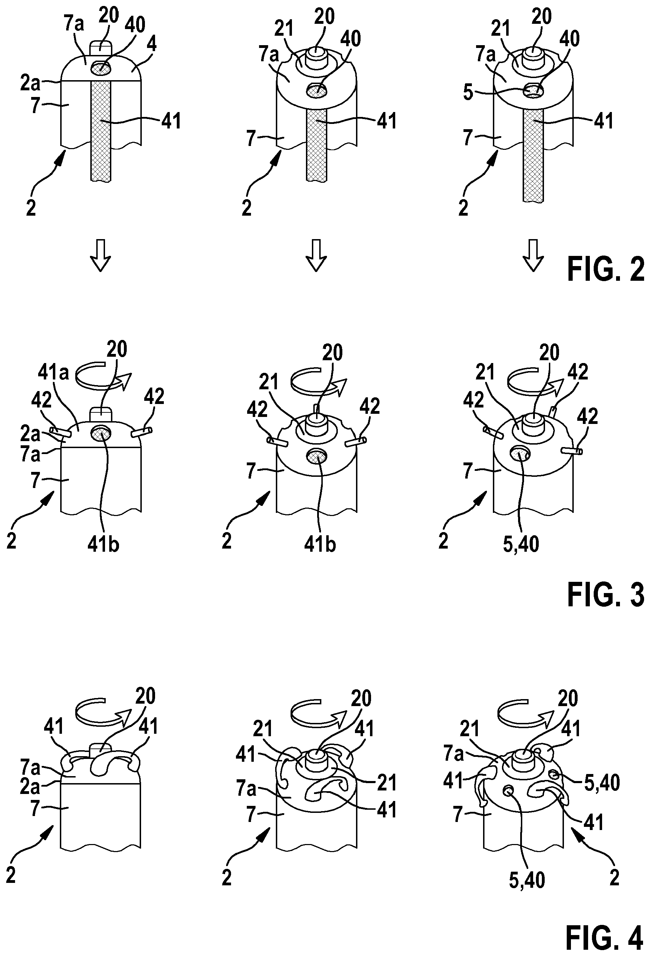

Further variants are captured in Figs. 2 to 4 where various alternative adhesive deployment strategies progressing forward from left to right are depicted that include a catheter-enabled ribbon-based deployment strategy (cf. Fig. 2), a spice-dispenser methodology (cf. Fig. 3), and a pop top configuration (cf. Fig. 4). Particularly, the latter two strategies depend on an instated implant rotation and the resulting mechanical interactions between structures of the surface of the distal end portion 7a of the casing 7 of the implant 2 and patient anatomy.

Particularly, as shown in Fig. 2, the at least one outlet 40 is closed by means of an elongated flexible (e.g. ribbon-like) closure 41 that is configured to be pulled from a first position in which the at least one outlet 40 is closed by the closure 41 into a second position in which the at least one outlet 40 is open.

Alternatively, as shown in Fig. 3, the implantable medical device 2 can comprises a rotatable closure 41 that closes the at least one outlet 40 by covering and therewith sealing the latter, wherein the rotatable closure 41 is configured to be rotated with respect to the casing 7 of the implantable medical device 2 to open the at least one outlet 40, wherein due to said rotation, a through-opening 41b of the closure 41 is aligned with the at least one outlet 40 so that the latter is now open. Particularly, the rotatable closure 41 comprises protrusions 42 protruding from a surface 41a of the rotatable closure 41, which protrusions 42 are configured to contact tissue of the patient at the implantation site so that the rotatable closure 41 can be rotated with respect to the casing 7 of the implantable medical device 2 by rotating the casing 7 when the protrusions 42 contact said tissue.

Furthermore, according to the alternative embodiment shown in Fig. 4 the implantable medical device 2 comprises one or several outlets, wherein each outlet is closed by means of an elongated pivotable closure 41 that is configured to be pivoted with respect to the casing 7 of the implantable medical device 2 away from the respective outlet to open the at respective outlet 40. Particularly, the respective pivotable closure 41 is configured to engage tissue 6 of the patient at the implantation site so that the respective pivotable closure 41 can be pivoted with respect to (and or torn from) the casing 7 of the implantable medical device 2 once the casing 7 is rotated and the pivotable closures 41 contact said tissue.

Further, the push-breach methodology described in conjunction with Fig. 1 may be combined with a catheter-based means for rupturing the adhesive reservoir 4 that could leverage the application of push-pull sliders in a handle of the catheter 3 that would force one or more sharp elements into the rupturable wall of the reservoir 4 to instate deployment of the adhesive 5.

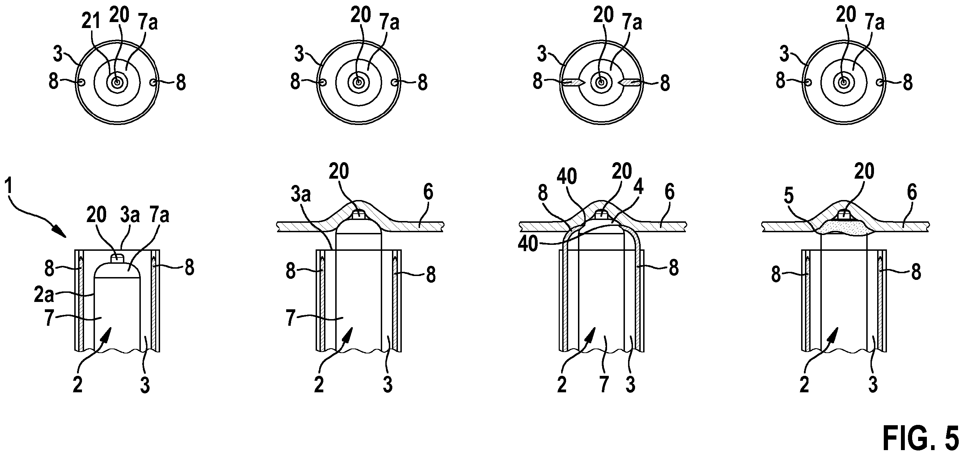

Particularly, Fig. 5 shows an embodiment of a medical implant delivery device and process of adhesive discharge from left to right, wherein the catheter 3 comprises two displaceable puncturing members 8 that are configured to be displaced along the longitudinal axis of the catheter 3 towards the distal end 3a of the catheter, in order to puncture the distal end portion 7a of the casing 7 of the implant 2 to form a corresponding number of outlets 40 of the reservoir 4 through which said liquid adhesive 5 is dischargeable onto the medical device 2 and/or onto the tissue 6 of the patient to anchor the implant 2.

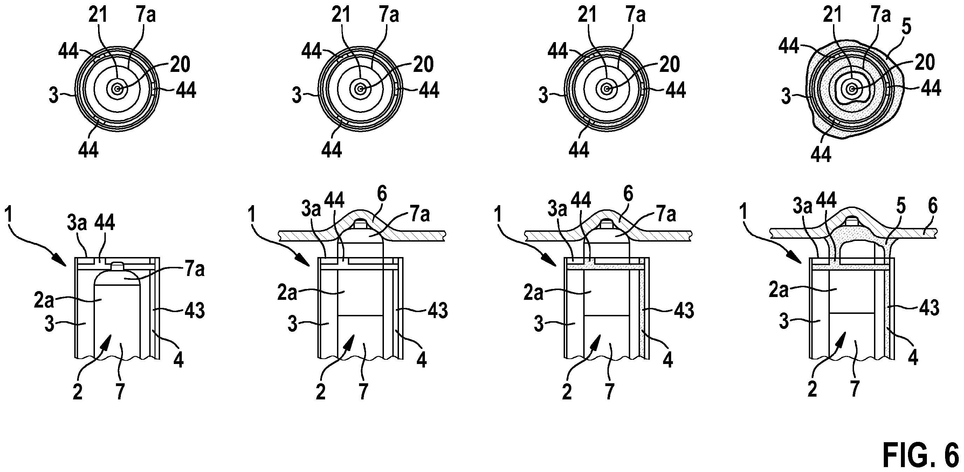

Additionally, Fig. 6 highlights an alternative catheter-based embodiment that employs the catheter to dispense the adhesive directly. Particularly, here, the catheter 3 comprises at least one channel 43 extending along the longitudinal axis of the catheter and forming at least a portion of said reservoir 4, wherein the at least one channel 43 comprises at least one opening 44 at the distal end 3a of the catheter 3 to discharge liquid adhesive 5 onto the distal end portion 2a of the implantable medical device 2 and/or onto tissue 6 of the patient at the implantation site to bond the implantable medical device 2 to said tissue 6.

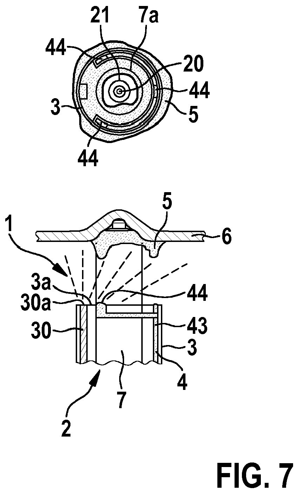

Further, Fig. 7 represents an extension of the embodiment shown in Fig. 6 which further details an adaptation for the curing of the adhesive enabled via UV excitation from a catheter-integrated fiber optic. As noted prior, such an approach is viable for pairing with all embodiments considered in this disclosure.

Particularly, here, the catheter 3 comprises an optical fiber 30 having an end portion 30a arranged at the distal end 3a of the catheter 3 to irradiate said applied adhesive 5 with UV light coupled into the optical fiber 30 so as to cure said applied liquid adhesive 5.

Additionally to all the above described embodiments, the implantable medical device 2 may comprise additional temporary anchoring means to temporarily anchor the device 2 to the myocardium. This would temporarily support the adhesive anchoring during the curing process, or, until the adhesive anchoring has settled otherwise. This temporary anchoring can comprise tines or sutures, which are degradable. This temporary anchoring may last at least until the implantable medical device 2 is encapsulated.

The present invention may have several advantages. Particularly, the implant 2 can be used to map the heart/implantations site before deploying its anchoring means, the liquid adhesive 5, as no sharp elements are presented to patient physiology at the implant's 2 distal terminus.

Further, the concept according to the present disclosure offers potential to eliminate the need for tether support in the implantation catheter, offering a means for reduced complexity and cost in the device's support infrastructure.

Furthermore, added flexibility for anchoring the device in a greater range of patient anatomical environments including thin-walled tissue structures where conduction pathways may prove optimal for device/patient electrical interactions, for example, being forced to anchor a device in the atrial appendage where mechanical fixation proves easier, but therapy support is challenged.

Furthermore, the invention offers an avenue to reduce the risk for thin-wall tissue perforation including the risk for cardiac tamponade.

Further, the invention offers an avenue to reduce the risk for unintended implant dislocation between follow-up.

Furthermore, the invention offers an avenue for potentially reducing necrotic tissue around the implant's electrical interface, potentially lowering the pacing capture thresholds and thereby improving product longevity in highly space constrained implants that depend upon primary cell power support.

Finally, the invention offers a means for viable anchoring of leadless/intracardiac pacemakers in the right atrium making multi-chamber leadless therapies much more approachable.

## Claims

1. A medical device delivery system (1), comprising:
- an implantable medical device (2),
- a delivery device (3) for delivering the implantable medical device (2) to an implantation site within a body of a patient,
wherein the medical device delivery system (1) comprises a reservoir (4) for accommodating an adhesive (5) in a liquid state, wherein the medical device delivery system (1) is configured to discharge the liquid adhesive (5) from the reservoir (4) so as to bond the implantable medical device to tissue (6) of the patient at the implantation site.

2. The medical device delivery system according to claim 1, wherein the reservoir (4) is comprised by the implantable medical device (2).

3. The medical device delivery system according to claim 1 or 2, wherein the implantable medical device (2) comprises a distal end portion (2a), wherein the reservoir (4) is arranged in or at the distal end portion (2a) of the implantable medical device (2).

4. The medical device delivery system according to claim 3, wherein for discharging said adhesive (5) the reservoir (4) is fluidly connected to at least one openable outlet (40) formed in a distal end portion (7a) of a casing (7) of the implantable medical device (2).

5. The medical device delivery system according to claim 4, wherein the at least one outlet (40) is configured to be opened by applying a pressure on said distal end portion (7a) of the casing (7).

6. The medical device delivery system according to claim 4, wherein the at least one outlet (40) is closed by means of an elongated flexible closure (41) that is configured to be pulled from a first position in which the at least one outlet (40) is closed by the closure (41) into a second position in which the at least one outlet (40) is open.

7. The medical device delivery system according to claim 4, wherein the implantable medical device (2) comprises a rotatable closure (41) that closes the at least one outlet (40), wherein the rotatable closure (41) is configured to be rotated with respect to the casing (7) of the implantable medical device (2) to open the at least one outlet (40).

8. The medical device delivery system according to claim 4, wherein the implantable medical device (2) comprises an elongated pivotable closure (41) that closes the at least one outlet (40), wherein the pivotable closure (41) is configured to be pivoted with respect to the casing (7) of the implantable medical device (2) to open the at least one outlet (40).

9. The medical device delivery system according to claim 3, wherein the delivery device (3) comprises at least one displaceable puncturing member (8) that is configured to be displaced so as to puncture the distal end portion (7a) of the casing (7) to form at least one outlet (40) of the reservoir (4) through which said liquid adhesive (5) is dischargeable.

10. The medical device delivery system according to claim 1, wherein the delivery device (3) comprises at least one channel (43) forming at least a portion of said reservoir (4), wherein the at least one channel (43) comprises at least one opening (44) at a distal end (3a) of the delivery device (3) to discharge liquid adhesive (5) on the distal end portion (2a) of the implantable medical device (2) and/or on tissue (6) of the patient at the implantation site to bond the medical implant device (2) to said tissue (6).

11. The medical device delivery system according to any one of the preceding claims, wherein the delivery device (3) comprises an optical fiber (30) having an end portion (30a) arranged at the distal end (3a) of the delivery device (3) to irradiate said applied adhesive (5) with UV light coupled into the optical fiber (30) so as to cure said applied liquid adhesive (5).

12. The medical device delivery system according to one of the preceding claims, wherein the implantable medical device (2) is an intracardiac pacemaker, wherein the pacemaker (2) comprises a pacing electrode (20).

13. The medical device delivery system according to claims 4 and 12, wherein the casing (7) comprises a circumferential protrusion (70) that protrudes from a surface (7c) of the casing (7) and extends along the pacing electrode (20), wherein the circumferential protrusion (70) is arranged between the pacing electrode (20) and the at least one outlet (40) to hinder liquid adhesive (5) from contacting the pacing electrode (20) upon discharging of the liquid adhesive (5) through the at least one outlet (40).

14. An implantable medical device (2) comprising a reservoir (4) for accommodating an adhesive (5) in a liquid state, wherein the implantable medical device (2) is configured to discharge the liquid adhesive (5) from the reservoir (4).

15. A method for bonding an implantable medical device (2) to tissue (6) of a patient at an implantation site using a medical device delivery system (1) according to one of the preceding claims, wherein the method comprises the steps of:
- positioning the implantable medical device (2) at the implantation site using the delivery device (3), and
- discharging liquid adhesive (5) out of the reservoir (4) to bond the implantable medical device (2) to the tissue (6).
